# EUROPEAN PATENT SPECIFICATION

(11) **EP 4 498 891 B1**
(45) Date of publication and mention of the grant of the patent: **31.12.2025**
(21) Application number: 23714860.6
(22) Date of filing: 29.03.2023
(51) Int. Cl.: A61B 3/00

(54) **ILLUMINATOR END PART**
LEUCHTENENDSTÜCK
PARTIE EXTRÉMITÉ D'ILLUMINATEUR

(30) Priority: 30.03.2022 NL 2031446
(43) Date of publication of application: 05.02.2025
(73) Proprietor: Crea IP B.V., 3237 MG Vierpolders (NL)
(72) Inventor: OKKERSE, Jan Wijnand, 3237 MG Vierpolders (NL)
(74) Representative: Nederlandsch Octrooibureau
(86) International application number: PCT/NL2023/050161
(87) International publication number: WO 2023/191624

(56) References cited:
- CH-A2- 710 150
- CN-A- 114 176 900
- US-A1- 2013 079 598
- US-A1- 2016 128 561
- US-A1- 2020 360 104

## Description

### Field of the invention

The present invention relates to an assembly of an ophthalmic illuminator instrument and an illuminator end part.

### Background art

US patent publication US5,582,608 discloses a lamellar illumination apparatus for eye surgery. A support fixture is provided with a light emitter, and directs light to a surgical field tangentially to the cornea, at an angle of between 0 to 45 degrees to the plane of the eye iris. The light entering the eye travels along the lamellae of the cornea, and as a result very little light reaches the back of the eye or is directed towards a surgical microscope causing glare. The light emitter may be mounted on or incorporated in an eyelid speculum or a fixation ring.

US patent publication US2021/0177393 discloses a trans-scleral illumination system for vitreoretinal surgery, having a speculum with two arms with blades at distal ends thereof. Optical fibers are present running through the arms with one or more end points located within the blades. The position and orientation of the end faces of the fibers determine the direction of light from the system

US 2013/079598 A1 discloses an endoilluminator system 10 for projecting light into an interior of an eyeball (paragraph 14). The system includes a cannula 20 having a substantially cylindrical shape that defines an interior region 30 with a cylindrical axis 32 (paragraph 15). Intermediate material 24 is disposed within interior region 30 and defines a fiber pathway 36 within which optical fiber 26 is disposed (paragraph 17). The fiber pathway 36 is curved so optical fiber 26 directs light at an angle θ from axis 32 as optical fiber 26 protrudes from fiber pathway 36 (paragraph 17). The angle θ may have values in ranges of less than 30, 30 to 60, 60 to 90, or 90 to 120 degrees (paragraph 17). Relief 34 may be shaped into cannula 20 to avoid vignetting or blocking emitted light (paragraph 18). The distal end 40 of optical fiber 26 may be shaped to direct the emitted light, including compound parabolic concentrator or tapered shape (paragraph 21).

US 2016/128561 A1 discloses an optical tomography device comprising an optical probe 50 for applying measurement light to a measurement target eye 200 (paragraphs 25-26). The distal end of the optical probe 50 can be inserted into an eyeball (paragraph 31). The optical probe 50 includes a probe housing 34 which is a cylindrical member whose distal end is closed and which protrudes from a rotary unit 32 (paragraph 36). On the side face of the probe housing 34, an open window 38 is formed in the vicinity of the distal end (paragraph 36). In the probe housing 34, a reflective mirror 36 is disposed at a position corresponding to the open window 38, with the reflection surface of the reflective mirror 36 being inclined by 45 degrees relative to the axis of the optical probe body 40 (paragraph 37). Light from the optical fiber is reflected at the reflection surface of the reflective mirror 36 to be applied to the outside of the probe housing 34 through the open window 38, with the light reflected by the reflective mirror 36 being applied in a direction orthogonal to the axis of the optical probe body 40 (paragraph 37).

CN 114 176 900 A discloses a scleral depressor with aiming light comprising an energy storage section 11, a rolling section 12, a tapered diameter section 13, and integrally formed connecting neck section 21 and elbow section 23 (background section and detailed description). The energy storage section 11 has a battery 111 fixed inside (detailed description). The rolling section 12 has a rolling member 122 with a laser emission device 1221 fixed on the side close to the connecting neck section 21. The tapered diameter section 13 is detachably and fixedly connected to the connecting neck section 21, and a reflecting mirror 221 is obliquely fixed on the inner surface of the rounded portion 22. Light emitted by the laser emission device 1221 enters the connecting neck section 21, is refracted by the reflecting mirror 221, enters the elbow section 23, and is then emitted.

US 2020/360104 A1 discloses a device 2 for an ophthalmological illumination system comprising a light instrument 3 for illuminating the intraocular space of a human or animal eye (paragraph 4). The device comprises a housing 4 having a proximal housing end 5, a distal housing end 6, and an opening 7 in the proximal housing end 5 (paragraph 4). The housing delimits a receptacle space 8 which extends from the opening 7 in the proximal housing end 5 along a longitudinal direction in the direction of the distal housing end 6, and is configured for receiving the light instrument 3 through the opening 7 (paragraph 4). The housing 4 comprises at least one translucent material at least in the region of the distal housing end 6 (paragraph 4). A very uniform light distribution of the light emitted by the light instrument is achieved by virtue of the housing comprising translucent material, which brings about a high degree of scattering and reflection of impinging light, and light reflections that disturb the user during use are avoided as a result (paragraph 5). The device 2 is releasably connectable to the light instrument 3, with the proximal region 12 of the housing 4 being configured to releasably connect to the distal region 28 of the handle 24 through positively locking and/ or force-locking connection (paragraphs 20, 58). **Summary of the invention**

The present invention seeks to provide a simple add-on design feature to existing ophthalmological instruments, such as (endo-)illuminators or laser probes, which allow to re-direct light in a desired direction different from the direction of light provided by the instrument itself.

According to the present invention, an Illuminator end part as defined above is provided, the Illuminator end part comprising an elongated sleeve part having a bore extending in a first direction with a diameter corresponding to an outside diameter of a light guiding end part of the ophthalmic illuminator instrument, and a light deflection part arranged to guide light originating from the light guiding end part in a direction away from the first direction.

### Short description of drawings

The present invention will be discussed in more detail below, with reference to the attached drawings, in which
Fig. 1 shows a perspective view of an embodiment of the Illuminator end part according to the present invention;
Fig. 2 shows a cross sectional view of the Illuminator end part shown in Fig. 1 attached to an ophthalmic illuminator instrument;
Fig. 3 shows a detailed cross sectional view of a tip of the Illuminator end part of Fig. 1; and
Fig. 4 shows a view of the combination of Illuminator end part and ophthalmic illuminator instrument during actual use.

### Description of embodiments

The present invention embodiments provide a simple and cost-efficient add-on functionality to ophthalmological instruments, such as (endo-)illuminators, which allow a broader spectrum of use of these instruments by providing a suitable re-direction of light from the instrument.

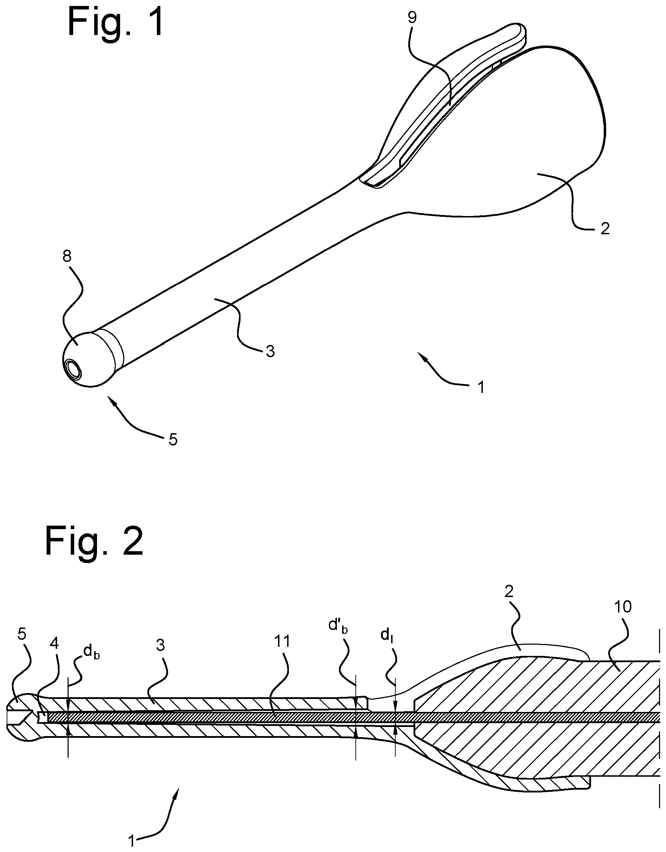

Fig. 1 shows a perspective view of an embodiment of the Illuminator end part 1 according to the present invention, comprising an (optional) instrument adapter part 2 allowing to attach the Illuminator end part 1 to attach to an ophthalmic illuminator instrument 10 (see Fig. 2 below), an elongated sleeve part 3 and a light deflection part 5. In other words, in a first group of embodiments, the Illuminator end part 1 comprises an instrument adaptor part 2, an elongated sleeve part 3 having a bore 4 extending in a first direction (i.e. a. longitudinal direction of the elongated sleeve part 3) with a diameter corresponding to an outside diameter of a light guiding end part 11 of the ophthalmic illuminator instrument 10, and a light deflection part 5 arranged to guide light originating from the light guiding end part 11 in a direction away from the first direction. This allows to have light exiting the combined ophthalmic illuminator instrument 10 and Illuminator end part 1 in a direction different from the regular direction of light of the ophthalmic illuminator instrument 10, i.e. in a longitudinal direction. In general, various directions, ranges and opening angles may be provided for the light exiting the light deflection part 5 of the Illuminator end part 1.

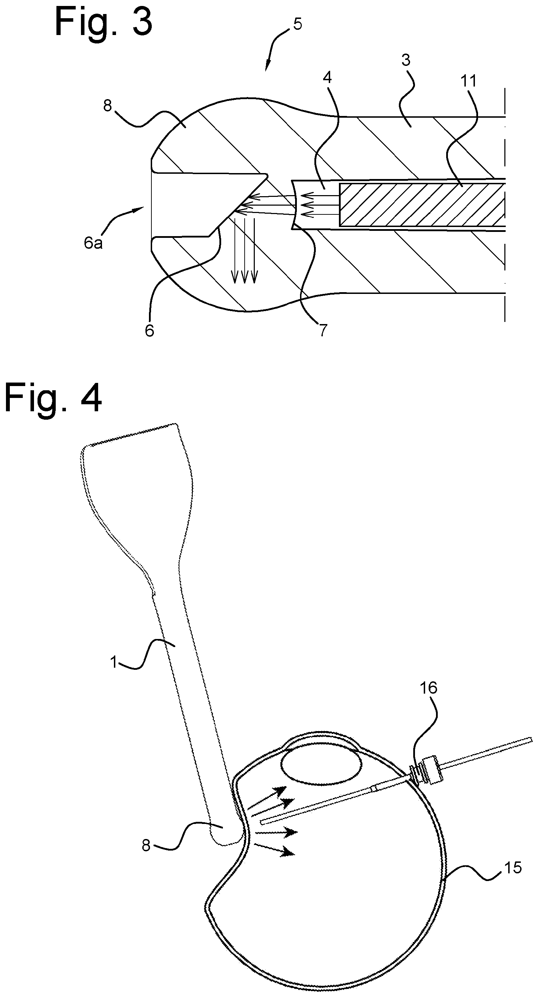

In a further embodiment, as shown in Fig. 1, the light deflection part 5 comprises an outer surface 8 for scleral depression, wherein the outer surface 8 has a maximum outer diameter larger than an outside diameter of the elongated sleeve part 3. The outer surface 8 on the light deflection part 5 provides an additional functionality to the combination of ophthalmic illuminator instrument 10 and Illuminator end part 1, as it is possible for the surgeon to use the outer surface 8 to put pressure on the outside of the eye. This allows to obtain an instrument in the form of an illuminated scleral depressor, which in combination with the ophthalmic illuminator instrument 10 provides a surgeon with the advantage of e.g. absolute control of the eye during peripheral vitrectomy as it allows for visualization of the peripheral retina by depression, as shown in Fig. 4.

Fig. 4 shows a view of the combination of Illuminator end part 1 and ophthalmic illuminator instrument 10 of Fig. 1 during actual use. In Fig. 4 a cross section is shown of an eye 15, wherein the eye globe is pushed inwardly using the outer surface 8 of the light deflection part 5, thus bringing the corresponding part of the inside surface of the eye globe within the surgeon's field of view (directly through the cornea, or through a microscope. At the same time, this area is illuminated from the outside of the eye globe via the light deflection part 5. E.g. using as indicated a trocar 16 and vitrectome 17, the indenting of the eye globe and endo-illumination allows the tissue of the globe to be analysed and e.g. to perform a precise shaving action using the vitrectome 17.

The light deflection function of the Illuminator end part 1 will be explained in further detail with reference to Fig. 2, which shows a cross sectional view of the Illuminator end part 1 shown in Fig. 1, attached to an ophthalmic illuminator instrument 10 provided with a light guiding end part 11, as shown with an outer diameter *dₗ*. along its entire length. In the exemplary embodiment shown, the bore 4 in the elongated sleeve part 3 tapers from an instrument adapter part side (first inner diameter *d_{b}'*) towards a light deflection part side (second inner diameter *d_{b}*). This ensures an easy entry of the light guiding end part 11 into the bore 4 of the Illuminator end part 1, and even a self-centring of the ophthalmic illuminator instrument 10 toward the light deflection elements in the light deflection part 5 in the tip of the Illuminator end part 1. The second inner diameter *d_{b}* near (or proximal to) the light deflection part 5 may be equal to or smaller than the outer diameter *dₗ* of the light guiding end part 11. In one group of embodiments, the second inner diameter *d_{b}* is substantially equal to the outer diameter *dₗ*, or alternatively, the second inner diameter *d_{b}* is smaller than the outer diameter *dₗ* as the tapering inner diameter of the bore 4 still allows proper alignment of various gauge light guiding elements 11.

Fig. 3 shows a detailed cross sectional view of a tip of an exemplary embodiment of the Illuminator end part of Fig. 1. In this exemplary embodiment, the light deflection part 5 comprises a deflection surface 6. Light exiting from an end surface of the light guiding end part 11 is deflected away from the longitudinal direction of the Illuminator end part 1 by the deflection surface 6.

In the embodiment shown, the deflection surface 6 is at an angle of about 45 degrees to the first direction (longitudinal direction of the Illuminator end part 1). This may be implemented by providing a properly formed void 6a in the light deflection part 5 as shown in Fig. 3. The deflection surface 6 will eventually cause a diversion or deflection of the light at 90 degrees from the first direction, which is very efficient to obtain a large amount of light into an eye during use (e.g. as shown in Fig. 4). In further embodiments, the deflection surface 6 may be at a different angle, or even have an optically formed surface, e.g. to scatter light in a (limited) range of directions.

In a further embodiment, the Illuminator end part 1 further comprises a light conversion member 7 aligned in the bore 4, wherein the light conversion member 7 is arranged to focus light originating from the light guiding end part 11 onto the deflection surface 6 during operation. This would allow to obtain a larger part of the light emanating from the light guiding end part 11 to eventually exit or end up out of the light deflection part 5. E.g. the light conversion member 7 may be implemented as a concave lens surface, in more general wording, the light conversion member 7 is a focussing surface provided as part of the light deflection part 5 in a further embodiment. Such a focussing surface may be easily obtained by properly shaping a surface formed in the material of the Illuminator end part 1. In addition or alternatively, the light conversion member 7 is provided as a (separate) lens element. This would allow an even more precise definition of the light beam eventually exiting the light deflection part 5 of the Illuminator end part 1. The light conversion member 7 in the form of a lens element can also be part of the light guiding end part 11.

In Fig. 3, the arrows show the light direction in the various regions of the light deflection part 5 during operation, in this example transitioning from a parallel beam exiting the light guiding end part 11 to a converging beam between light conversion member 7 and deflection surface 6, to again a parallel beam between the deflection surface 6 and outside surface 8.

In order to properly guide light, the light deflection part 5 comprises an opaque or transparent material. In an exemplary further embodiment, the opaque or transparent material is one of a medical grade acrylonitrile butadiene styrene (ABS). poly methyl methacrylate (PMMA) or glass. E.g. ABS is sufficiently strong to allow the Illuminator end part 1 to be used as deflector, and provides a sufficiently high transparency to allow the light energy to exit from the Illuminator end part 1.

In a further embodiment, the opaque or transparent material has a refractive index higher than 1. This allows the deflection surface 6 to be formed by a material-air interface which is easy to realize using well known manufacturing techniques such as injection moulding. The Illuminator end part 1 as shown in the Fig. 1-4 embodiments can be manufactured easily using injection moulding techniques.

As shown in Fig. 4, an advantageous use of the present invention embodiments is to use the Illuminator end part 1 also for depression of (scleral) tissues. In order to safely and accurately use the Illuminator end part 1, the outer surface 8 has a continuously varying outer diameter in a further embodiment. This ensures no sharp transitions or edges are present which could damage tissue during use. In an even further embodiment, the outer surface 8 has a droplet shape, e.g. comprising a smooth convex outer surface, aiding in providing simple and effective use of the depression function of the Illuminator end part 1.

The present invention Illuminator end part 1, in a further embodiment comprises an instrument adapter part 2, allowing the illuminator end cap 1 to be attached to its associated ophthalmic instrument using the instrument adapter part 2. In an exemplary embodiment, the instrument adapter part 2 has a circumferential surface with a slit 9 in the first direction. The slit 9 in the instrument adapter part 2 is easy to manufacture (e.g. using injection moulding), and provides for a robust attachment to the associated instrument. Furthermore, the slit 9 allows a more easy introduction and alignment of the light guiding end part 11 (usually a fiber) with the bore 4, which prevents possible damage to the light guiding end part 11 while assembling. In addition, as the deflection surface 6 and slit 9 can have a fixed mutual orientation, the slit 9 may also act as a user indicator for the light emission direction of the Illuminator end part 1 (e.g. at 0 degree or at 180 degrees).

Furthermore, in a further group of embodiments (most embodiments shown in the figures and described above), the Illuminator end part 1 is a single piece component. The Illuminator end part 1 may be manufactured by injection moulding, which allows for cost-effective and material effective manufacturing, which may also allow the Illuminator end part 1 to be a single use, disposable item.

## Claims

1. An assembly of an ophthalmic illuminator instrument (10) and an illuminator end part (1), the illuminator instrument (10) comprising an elongate light guiding end part (11), extending in a first direction from an instrument side towards a light deflection part side,
the illuminator end part (1) comprising:
- an elongated sleeve part (3) having a bore (4) extending in a first direction with a diameter corresponding to an outside diameter of a light guiding end part (11) of the ophthalmic illuminator instrument (10), and tapering from an instrument side towards a light deflection part side,
- a light deflection part (5) arranged to guide light originating from the light guiding end part (11) in a direction away from the first direction,
- an instrument adapter part (2) that is releasably connected to the illuminator instrument.

2. The assembly according to claim 1, wherein the light deflection part (5) comprises a deflection surface (6).

3. The assembly according to claim 2, wherein the deflection surface (6) is at an angle of about 45 degrees to the first direction.

4. The assembly according to claim 2 or 3, further comprising a light conversion member (7) aligned in the bore (4), wherein the light conversion member (7) is arranged to focus light originating from the light guiding end part (11) onto the deflection surface (6) during operation.

5. The assembly according to claim 4, wherein the light conversion member (7) is a focussing surface provided as part of the light deflection part (5).

6. The assembly according to claim 4, wherein the light conversion member (7) is a lens element.

7. The assembly according to any one of claims 1-6, wherein the light deflection part (5) comprises an opaque or transparent material.

8. The assembly according to claim 7, wherein the opaque or transparent material is medical grade acrylonitrile butadiene styrene (ABS), poly methyl methacrylate (PMMA) or glass.

9. The assembly according to any one of claims 1-8, wherein the light deflection part (5) comprises an outer surface (8) for scleral depression, wherein the outer surface (8) has a maximum outer diameter larger than an outside diameter of the elongated sleeve part (3).

10. The assembly according to claim 9, wherein the outer surface (8) has a continuously varying outer diameter.

11. The assembly according to claim 9 or 10, wherein the outer surface (8) has a droplet shape.

12. The assembly according to any one of claims 1-11, wherein the instrument adapter part (2) has a circumferential surface with a slit (9) in the first direction.

13. The assembly according to any one of claims 1-12, wherein the illuminator end part (1) is a single piece component.

## Patentansprüche

1. Eine Anordnung aus einem ophthalmologischen Beleuchtungsinstrument (10) und einem Beleuchtungsendteil (1), wobei das Beleuchtungsinstrument (10) ein längliches lichtleitendes Endteil (11) umfasst, das sich in einer ersten Richtung von einer Instrumentenseite zu einer Lichtablenkungsteilseite erstreckt, wobei das Beleuchtungsendteil (1) umfasst:
- ein längliches Hülsenteil (3) mit einer Bohrung (4), die sich in einer ersten Richtung erstreckt und einen Durchmesser aufweist, der dem Außendurchmesser eines lichtleitenden Endteils (11) des ophthalmologischen Beleuchtungsinstruments (10) entspricht, und sich von einer Instrumentenseite zu einer Lichtablenkungsteilseite hin verjüngt,
- ein Lichtablenkungsteil (5), das so angeordnet ist, dass es vom lichtleitenden Endteil (11) ausgehendes Licht in einer von der ersten Richtung abweichenden Richtung leitet,
- ein Instrumentenadapterteil (2), das lösbar mit dem Beleuchtungsinstrument verbunden ist.

2. Die Anordnung gemäß Anspruch 1, wobei das Lichtablenkungsteil (5) eine Ablenkungsfläche (6) umfasst.

3. Die Anordnung gemäß Anspruch 2, wobei die Ablenkungsfläche (6) in einem Winkel von etwa 45 Grad zur ersten Richtung steht.

4. Die Anordnung gemäß Anspruch 2 oder 3, die ferner ein Lichtumwandlungselement (7) umfasst, das in der Bohrung (4) ausgerichtet ist, wobei das Lichtumwandlungselement (7) so angeordnet ist, dass es während des Betriebs Licht, das vom lichtleitenden Endteil (11) stammt, auf die Ablenkungsfläche (6) fokussiert.

5. Die Anordnung gemäß Anspruch 4, wobei das Lichtumwandlungselement (7) eine Fokussierfläche ist, die als Teil des Lichtablenkungsteils (5) vorgesehen ist.

6. Die Anordnung gemäß Anspruch 4, wobei das Lichtumwandlungselement (7) ein Linsenelement ist.

7. Die Anordnung gemäß einem der Ansprüche 1 bis 6, wobei das Lichtablenkungsteil (5) aus einem lichtundurchlässigen oder transparenten Material besteht.

8. Die Anordnung gemäß Anspruch 7, wobei das lichtundurchlässige oder transparente Material medizinisches Acrylnitril-Butadien-Styrol (ABS), Polymethylmethacrylat (PMMA) oder Glas ist.

9. Die Anordnung gemäß einem der Ansprüche 1 bis 8, wobei das Lichtablenkungsteil (5) eine Außenfläche (8) zur Skleraldepression umfasst, wobei die Außenfläche (8) einen maximalen Außendurchmesser aufweist, der größer ist als der Außendurchmesser des länglichen Hülsenteils (3).

10. Die Anordnung gemäß Anspruch 9, wobei die Außenfläche (8) einen sich kontinuierlich ändernden Außendurchmesser aufweist.

11. Die Anordnung gemäß Anspruch 9 oder 10, wobei die Außenfläche (8) eine Tropfenform aufweist.

12. Die Anordnung gemäß einem der Ansprüche 1 bis 11, wobei das Instrumentenadapterteil (2) eine Umfangsfläche mit einem Schlitz (9) in der ersten Richtung aufweist.

13. Die Anordnung gemäß einem der Ansprüche 1 bis 12, wobei das Beleuchtungsendteil (1) ein einteiliges Bauteil ist.

## Revendications

1. Ensemble composé d'un instrument d'éclairage ophtalmique (10) et d'une partie extrémité d'illuminateur (1), l'instrument d'éclairage (10) comprenant une partie extrémité de guidage de lumière allongée (11), s'étendant dans une première direction à partir d'un côté instrument vers un côté de partie de déviation de lumière,
la partie extrémité d'illuminateur (1) comprenant :
- une partie de manchon allongée (3) ayant un alésage (4) s'étendant dans une première direction avec un diamètre correspondant à un diamètre extérieur d'une partie extrémité de guidage de lumière (11) de l'instrument d'éclairage ophtalmique (10) et se rétrécissant d'un côté instrument vers un côté de partie de déviation de lumière,
- une partie de déviation de lumière (5) agencée pour guider la lumière provenant de la partie extrémité de guidage de lumière (11) dans une direction s'éloignant de la première direction,
- une partie adaptateur d'instrument (2) qui est reliée de manière amovible à l'instrument d'éclairage.

2. Ensemble selon la revendication 1, où la partie de déviation de lumière (5) comprend une surface de déviation (6).

3. Ensemble selon la revendication 2, où la surface de déviation (6) forme un angle d'environ 45 degrés par rapport à la première direction.

4. Ensemble selon la revendication 2 ou 3, comprenant en outre un élément de conversion de lumière (7) aligné dans l'alésage (4), où l'élément de conversion de lumière (7) est agencé pour focaliser la lumière provenant de la partie extrémité de guidage de lumière (11) sur la surface de déviation (6) pendant le fonctionnement.

5. Ensemble selon la revendication 4, où l'élément de conversion de lumière (7) est une surface de focalisation fournie comme partie intégrante de la partie de déviation de lumière (5).

6. Ensemble selon la revendication 4, où l'élément de conversion de lumière (7) est un élément de lentille.

7. Ensemble selon l'une quelconque des revendications 1 à 6, où la partie de déviation de lumière (5) comprend un matériau opaque ou transparent.

8. Ensemble selon la revendication 7, où le matériau opaque ou transparent est de l'acrylonitrile butadiène styrène (ABS) de qualité médicale, du polyméthacrylate de méthyle (PMMA) ou du verre.

9. Ensemble selon l'une quelconque des revendications 1 à 8, où la partie de déviation de lumière (5) comprend une surface extérieure (8) pour la dépression sclérale, où la surface extérieure (8) a un diamètre extérieur maximal supérieur à un diamètre extérieur de la partie de manchon allongée (3).

10. Ensemble selon la revendication 9, où la surface extérieure (8) a un diamètre extérieur variant continuellement.

11. Ensemble selon la revendication 9 ou 10, où la surface extérieure (8) a une forme de gouttelette.

12. Ensemble selon l'une quelconque des revendications 1 à 11, où la partie adaptateur d'instrument (2) présente une surface circonférentielle avec une fente (9) dans la première direction.

13. Ensemble selon l'une quelconque des revendications 1 à 12, où la partie extrémité d'illuminateur (1) est un composant monobloc.
